(19) Europäisches Patentamt
European Patent Office
Office européen des brevets

(11) EP 1 424 060 B1

(12) FASCICULE DE BREVET EUROPEEN

(45) Date de publication et mention de la délivrance du brevet:
**07.02.2007 Bulletin 2007/06**

(51) Int Cl.:
*A61K 8/34* (2006.01) *A61K 8/27* (2006.01)
*A61K 8/19* (2006.01) *A61Q 5/10* (2006.01)
*D06B 11/00* (2006.01) *A23L 1/275* (2006.01)

(21) Numéro de dépôt: **03292517.4**

(22) Date de dépôt: **10.10.2003**

(54) **Agent de coloration des matières kératiniques humaines à au moins deux composants et procédés de coloration**

Mittel zum färben von menschlichem Keratinmaterial, enthaltend mindestens zwei Komponenten und Färbeverfahren

Agent for dyeing material containing human keratin, with at least two components and method for dyeing

(84) Etats contractants désignés:
**AT BE BG CH CY CZ DE DK EE ES FI FR GB GR HU IE IT LI LU MC NL PT RO SE SI SK TR**

(30) Priorité: **29.11.2002 FR 0215051**

(43) Date de publication de la demande:
**02.06.2004 Bulletin 2004/23**

(73) Titulaire: **L'ORÉAL**
**75008 Paris (FR)**

(72) Inventeurs:
- **Pruche, Francis**
**60300 Senlis (FR)**
- **Chevalier, Véronique**
**94440 Villecresnes (FR)**

(74) Mandataire: **Miszputen, Laurent**
**L'OREAL - D.I.P.I.**
**25-29 Quai Aulagnier**
**92600 Asnières (FR)**

(56) Documents cités:
**WO-A-02/30371** **WO-A-02/30375**
**US-A- 5 895 642** **US-A1- 2002 124 330**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention concerne d'une manière générale un agent de coloration des matières kératiniques à au moins deux composants, en particulier pour la coloration de la peau et/ou des fibres kératiniques humaines et divers procédés de coloration le mettant en oeuvre.

Dans le domaine de la coloration des matières kératiniques humaines telles que la peau, les cheveux, les cils, les sourcils et les poils, on utilise à l'heure actuelle des catalyseurs enzymatiques pour activer la coloration de précurseurs de coloration. Ainsi, on active la coloration de polyphénols par oxydation en présence de polyphénoloxydase naturelle. A titre d'exemple, de la catéchine, en présence de polyphénoloxydase naturelle, donne une coloration jaune orangée et les dihydroxyphénylanaline (L. DOPA) donne de la mélanine. L'avantage principal de ces catalyseurs enzymatiques consiste en l'obtention de pigments de couleurs et de nuances originales, sans utilisation de composés oxydants. Cependant, l'inconvénient majeur de ce procédé de coloration est l'utilisation d'enzymes, pour lesquels les risques toxicologiques, la stabilité dans les compositions, la reproductibilité, le prix et l'immobilisation souvent nécessaire sont des facteurs qui limitent grandement leurs applications.

D'autre part, ces catalyseurs sont de nature protéique et l'utilisation de protéines n'est pas sans risque pour une utilisation en cosmétologie ou en dermatologie, notamment en raison des réactions de sensibilisation.

L'utilisation de catalyseurs enzymatiques dans les préparations cosmétiques du type auto-bronzant ne permet pas toujours une coloration homogène de la peau. L'application sur l'ensemble du corps de compositions contenant de la dihydroxyacétone (ou DHA), typiquement utilisée dans ce type d'application, est longue et fastidieuse, et l'obtention d'une coloration homogène est difficile.

Dans le domaine des crèmes bronzantes et autobronzantes, une amélioration a été obtenue en utilisant à la place des catalyseurs enzymatiques, des catalyseurs chimiques. Ainsi, la demande WO 92/20321 A décrit une crème favorisant le brunissement de peaux claires lors d'une exposition au soleil ou à des rayons UVB, dont la composition comprend un milieu physiologiquement acceptable et une pseudocatalase. La pseudocatalase est un complexe de coordination d'un métal de transition dont le métal est Cu(I), Fe(II) ou Mn(II) et le ligand du bicarbonate. Par pseudocatalase, on entend un composé physiologiquement acceptable qui catalyse la dismutation de $H_20_2$ in vivo de manière analogue à une catalase.

Pour le traitement de la dépigmentation de la peau liée à des blocages de la transformation de la tyrosine en mélanine, comme par exemple le vitiligo, la demande WO92/20354 et le brevet US 5,895,642 décrivent des compositions contenant, dans un milieu physiologiquement acceptable, une pseudo-catalase.

Cette pseudo-catalase est un complexe de coordination de Fe(II), Cu(I) ou Mn(II), le ligand étant du bicarbonate.

L'article de K. Schallreuter (« Pseudocatalase is a bis-manganese III-EDTA-$(HC0_3)_2$ complex activated by UVB or natural sun ; J Investing Dermator Symp Proc 1999 Sep; 451; 91-6) mentionne l'utilisation d'un mélange d'hydrogénocarbonate de sodium et de manganèse ayant une activité pseudo-catalase pour le traitement du vitiligo. Cependant, il n'y a pas, dans l'ensemble de ces documents, d'indication concernant la coloration. D'autre part cette composition contient un chelateur qui est l'EDTA.

Dans le domaine de la coloration capillaire, le brevet européen EP 621.029 a décrit une composition comprenant du chlorite de sodium, un sel hydrosoluble de Fe, Mn ou Cu, ou un chélate de ce sel et un précurseur de colorant d'oxydation.

La coloration des cheveux nécessite l'emploi de combinaisons $H_2O_2$- ammonium ou amine.

On connaît également dans le brevet US 6,399,046 un procédé d' intensification du bronzage naturel de la peau qui consiste à stimuler in situ la mélanogénèse avec des polyphénols du type catéchine, ester gallique de catéchine ou des extraits de plantes contenant de la catéchine ou un ester gallique de catéchine, en particulier des extraits de feuille de thé vert. Ce procédé n'apporte pas de coloration directe et rapide sur la peau et la présence de mélanocytes est nécessaire.

On connaît également dans la demande US2002/0124330 des agents de coloration (A) des matières kératiniques comprenant au moins un précurseur de colorant d'oxydation du type amines aromatiques du type ortho ou para en présence d'oxygène, par oxydation au moyen d'un système catalytique purement chimique comprenant un premier constituant choisi parmi les sels et oxydes de Mn (II), et/ou Zn (II) et leurs mélanges et un second constituant choisi parmi les hydrogénocarbonates alcalins, les hydrogénocarbonates alcalino-terreux et leurs mélanges.

On connaît également dans la demande WO02/30371 des agents de coloration (A) des matières kératiniques comprenant au moins une enzyme propigmentante un acide aminé comportant un groupe thiol en présence d'oxygène, par oxydation au moyen d'un système catalytique purement chimique comprenant un premier constituant choisi parmi les sels et oxydes de Mn (II), et/ou Zn (II) et leurs mélanges et un second constituant choisi parmi les hydrogénocarbonates alcalins, les hydrogénocarbonates alcalino-terreux et leurs mélanges.

Il existe donc un besoin de trouver de nouvelles compositions de coloration des matières kératiniques, en particulier de coloration de la peau et / ou des fibres kératiniques, ne nécessitant pas l'utilisation de systèmes enzymatiques.

On a découvert dans la demande de brevet WO02/30375 qu'il était possible d'atteindre cet objectif en utilisant un agent de coloration (A) comprenant au moins un précurseur de colorant choisi parmi les composés comportant au moins un cycle aromatique ayant au moins deux groupes hydroxyle (OH) portés par deux atomes de carbone consécutifs du cycle aromatique et éventuellement un acide aminé comportant un groupe thiol, susceptible de se colorer en présence d'oxygène, par oxydation au moyen d'un système catalytique purement chimique comprenant un premier constituant choisi parmi les sels et oxydes de Mn (II), et/ou Zn (II) et leurs mélanges et un second constituant choisi parmi les hydrogénocarbonates alcalins, les hydrogénocarbonates alcalino-terreux et leurs mélanges. Ledit système catalytique chimique se comporte comme une pseudo-oxydase capable de mimer l'activité oxydase sans les inconvénients liés à l'emploi d'un système enzymatique.

Cependant, la Demanderesse a constaté d'une part que la persistance dans le temps, l'intensité et l'uniformité des couleurs obtenues par ce type d'agent de coloration (A) pouvaient être encore améliorées. D'autre part, ce type de procédé ne permet pas de contrôler suffisamment la réaction de coloration sur les cheveux ou sur la peau et d'obtenir une large gamme de teintes plus ou moins intenses selon les besoins de l'utilisateur et notamment à différents moments dans la journée ou sur une période de plusieurs jours.

La demanderesse a découvert de manière surprenante qu'en appliquant sur le support kératinique à colorer une deuxième composition acide (B) après l'application de l'agent de coloration (A) telle que défini précédemment , on stoppait la réaction de coloration révélée par ledit agent (A). Par un phénomène de mordançage, la composition acide (B) permettait de fixer les tannins ainsi formés sur les protéines des matières kératiniques. L'application de la composition acide (B) permettait de façon inattendue d'une part de renforcer la tenue de la couleur dans le temps et d'autre part de contrôler la réaction de coloration sur le support kératinique en la stoppant jusqu'à obtenir la teinte souhaitée par l'utilisateur.

La demanderesse a découvert également de façon surprenante qu'en appliquant une composition alcaline (C) après l'application de l'agent de coloration (A) telle que défini précédemment et éventuellement après application de la composition (B) acide définie précédemment, on pouvait raviver la couleur obtenue dans l'étape précédente en augmentant son intensité; la nouvelle teinte obtenue pouvant être à nouveau contrôlée en stoppant la réaction au moment choisi par application de ladite seconde composition (B).

Ce processus de coloration, fixation et/ou de ravivage de la couleur peut être renouvelé autant de fois que nécessaires et ce sur une durée de plusieurs jours.

La présente invention a donc pour objet deux procédés de coloration des matières kératiniques mettant en oeuvre un agent de coloration des matières kératiniques à au moins deux composants; caractérisé par le fait qu'il comprend

(i) un premier composant (A) unique ou à deux composants ($A_1$) et ($A_2$) comprenant, dans un milieu physiologiquement acceptable, au moins un précurseur de colorant choisi parmi les composés comportant au moins un cycle aromatique ayant au moins deux groupes hydroxyles portés par deux atomes de carbone consécutifs du cycle aromatique et au moins un système catalytique comprenant un premier catalyseur (1) choisi parmi les sels et oxydes de Mn(II) et/ou de Zn(II) et leurs mélanges et un second catalyseur (2) choisi parmi les hydrogénocarbonates alcalins, les hydrogénocarbonates alcalino-terreux et leurs mélanges ; les catalyseurs (1) et (2) pouvant être présents dans un seul constituant ou séparés dans deux composants ($A_1$) et ($A_2$) ;
(ii) un composant (B) comprenant une composition acide et/ou
(iii) un composant (C) comprenant une composition alcaline.

On entend par "matières kératiniques ", les fibres textiles naturelles constituées de kératine comme le coton, la soie, la laine et les matières comme la peau, le cuir chevelu, les ongles, les cheveux, les poils, les cils et les sourcils ainsi que les muqueuses.

Dans l'agent de teinture conforme à l'invention, le composant (A) peut être unique ou à deux composants ($A_1$) et ($A_2$). Il comprend au moins un précurseur de colorant choisi parmi les composés comportant au moins un cycle aromatique ayant au moins deux groupes hydroxyles portés par deux atomes de carbone consécutifs du cycle aromatique et au moins un système catalytique comprenant un premier catalyseur (1) choisi parmi les sels et oxydes de Mn(II) et/ou de Zn(II) et leurs mélanges et un second catalyseur (2) choisi parmi les hydrogénocarbonates alcalins, les hydrogénocarbonates alcalino-terreux et leurs mélanges ; les catalyseurs (1) et (2) pouvant être présents dans un seul composant ou séparés dans deux composants ($A_1$) et ($A_2$).

Selon un mode particulier de l'invention, le composant (A) tinctorial comprend deux composants ($A_1$) et ($A_2$) conditionnés séparément avec :

($A_1$) comprenant dans un milieu physiologiquement acceptable ledit précurseur de colorant et l'un des catalyseurs (1) ou (2) et ($A_2$) comprenant dans un milieu physiologiquement l'autre des catalyseurs (1) ou (2).

Les proportions du premier catalyseur (1) et du second catalyseur (2) sont de préférence choisies de telle sorte que :

$$\frac{[Mn(II)]}{[HCO_3]} \leq 1 \text{ avec } [\text{Mn(II)}] \neq 0$$

$$\frac{[Zn(II)]}{[HC0_3]} \leq 1 \text{ avec } [\text{Zn(II)}] \neq 0$$

$$\frac{[Mn(II) + Zn(II)]}{[HCO_3]} \leq 1 \text{ avec } [\text{Mn(II)}] \text{ et } [\text{Zn(II)}] \neq 0$$

où [Mn(II)], [Zn(II)] et [$HCO_3$] représentent respectivement les concentrations molaires en Mn(II), Zn(II) et $HCO_3$ dans la composition.

Généralement, le rapport $\frac{[Mn(II)]}{[HCO_3]}$ varie de $10^{-5}$ à $10^{-1}$, de préférence de $10^{-3}$ à $10^{-2}$ et est typiquement de l'ordre de $5.10^{-3}$.

Dans le cas de Zn(II), le rapport $\frac{[Zn(II)]}{[HCO_3]}$ est en général d'un ordre de 10 à 100 fois supérieur au rapport dans le cas de Mn(II).

Typiquement, ce rapport est de $10^{-4}$ ou plus, de préférence $10^{-3}$ ou plus, et de préférence de l'ordre de $5.10^{-1}$.

Dans le cas d'un mélange de Mn (II) et Zn (II), le rapport $\frac{[Mn(II) + Zn(II)]}{[HCO_3]}$ varie généralement de $10^{-5}$ à $10^{-1}$, de préférence $10^{-3}$ à $10^{-2}$, ce rapport étant choisi plus élevé lorsque la proportion de Zn (II) dans le mélange s'accroît.

Généralement, la concentration molaire en Mn (II), Zn (II), ou Mn (II) + Zn (II) dans la composition finale varie de $10^{-3}$ à 10 mM/l, de préférence de $10^{-2}$ à 1 mM/l.

Lorsqu'on utilise seulement un ou plusieurs sels ou oxydes de Mn (II), la concentration molaire en Mn(II) dans la composition finale est typiquement de $10^{-3}$ à $10^{-1}$ mM/l, de préférence $10^{-2}$ à $10^{-1}$ mM/l.

De préférence, lorsqu'on utilise uniquement un ou plusieurs sels ou oxydes de Zn(II), la concentration en Zn (II) dans la composition finale est de $5.10^{-2}$ à 10 mM/l, mieux de $5.10^{-1}$ à 1 mM/l.

Parmi les sels de Mn(II) et Zn(II) convenant pour la présente invention, on peut citer les chlorure, fluorure, iodure, sulfate, phosphate, nitrate et perchlorate, les sels d'acides carboxyliques et leurs mélanges. Ils peuvent provenir d'une eau minérale naturelle.

A titre d'exemple, on peut citer le chlorure de manganèse, le carbonate de manganèse (par exemple rhodochrosite), le difluorure de Mn (II), l'acétate de Mn(II) tétrahydraté, le lactate de Mn (II) trihydraté, le phosphate de Mn (II), l'iodure de Mn (II), le nitrate de Mn (II) trihydraté, le bromure de Mn (II) et le perchlorate de Mn (II) tétrahydraté, et le sulfate de Mn (II) monohydraté.

Les sels particulièrement préférés sont $MnCl_2$ et $ZnCl_2$.

Les sels d'acides carboxyliques incluent également des sels d'acides carboxyliques hydroxylés tels que le gluconate.

Parmi les hydrogénocarbonates alcalins et alcalino-terreux, on peut citer les hydrogénocarbonates de Na, K, Mg, Ca et leurs mélanges, préférentiellement l'hydrogénocarbonate de Na. Ils peuvent provenir d'une eau minérale naturelle.

Comme indiqué précédemment, le système catalytique chimique selon l'invention constitue une pseudo-oxydase en ce qu'il oxyde les polyphénols, en présence d'oxygène, comme ferait un catalyseur enzymatique naturel ayant une activité polyphénoloxydase.

Les précurseurs de colorant des compositions de l'invention sont des composés ou mélanges de composés comprenant au moins un cycle aromatique, de préférence un cycle benzénique, comportant au moins deux groupes hydroxyles (OH) portés par deux atomes de carbone consécutifs du cycle aromatique.

Le cycle aromatique peut être un cycle aromatique condensé contenant éventuellement un ou plusieurs hétéroatomes, tel que le naphtalène, le tétrahydronaphtalène, l'indane, l'indène, l'anthracène, le phénanthrène, l'indole, l'isoindole, l'indoline, l'isoindoline, le benzofuranne, le dihydrobenzofuranne, le chromane, l'isochromane, le chromène, l'isochromène, la quinoléine, la tétrahydroquinoléine et l'isoquinoléine.

Les précurseurs de colorant selon l'invention peuvent être représentés par la formule :

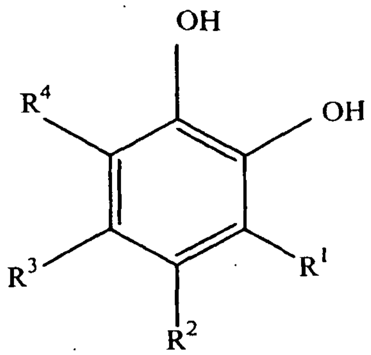

(I)

dans laquelle les substituts $R^1$ à $R^4$, identiques ou différents, représentent un atome d'hydrogène, un radical halogène, hydroxyle, carboxyle, carboxylate d'alkyle, amino éventuellement substitué, alkyle linéaire ou ramifié éventuellement substitué, alcényle linéaire ou ramifié éventuellement substitué, cycloalkyle éventuellement substitué, alcoxy, alcoxyalkyle, alcoxyaryle, le groupe aryle pouvant être éventuellement substitué, aryle, aryle substitué, un radical hétérocyclique éventuellement substitué, un radical contenant un ou plusieurs atomes de silicium, où deux des substituants $R^1$ à $R^4$ forment conjointement un cycle saturé ou insaturé contenant éventuellement un ou plusieurs hétéroatomes et éventuellement condensé avec un ou plusieurs cycles saturés ou insaturés contenant éventuellement un ou plusieurs hétéroatomes.

Les cycles saturés ou insaturés, éventuellement condensés, peuvent être aussi éventuellement substitués.

Les radicaux alkyles sont généralement les radicaux alkyles en $C_1$-$C_{10}$, de préférence les radicaux alkyles en $C_1$-$C_6$, tels que méthyle, éthyle, propyle, butyle, pentyle et hexyle.

Les radicaux alcoxy sont en général les radicaux alcoxy en $C_1$-$C_{20}$, tels que méthoxy, éthoxy, propoxy et butoxy.

Les radicaux alcoxy alkyles sont de préférence les radicaux alcoxy ($C_1$-$C_{20}$) alkyle ($C_1$-$C_{20}$), tels que méthoxyméthyle, éthoxyméthyle, méthoxyéthyle, éthoxyéthyle, etc.

Les radicaux cycloalkyles sont en général les radicaux cycloalkyles en $C_4$-$C_8$, de préférence les radicaux cyclopentyle et cyclohexyle. Les radicaux cycloalkyles peuvent être des radicaux cycloalkyles substitués, en particulier par des groupes alkyles, alcoxy, acide carboxylique, hydroxyle, amine et cétone.

Les radicaux alcényles sont de préférence des radicaux en $C_1$-$C_{20}$, tels que éthylène, propylène, butylène, pentylène, méthyl-2-propylène et décylène.

Les radicaux contenant un ou plusieurs atomes de silicium sont de préférence les radicaux polydiméthylsiloxane, polydiphenylsiloxane, polydiméthylphénylsiloxane, stéraoxydiméthicone.

Les radicaux hétérocycliques sont en général des radicaux comprenant un ou plusieurs hétéroatomes choisis parmi O, N et S, de préférence O ou N, éventuellement substitués par un ou plusieurs groupes alkyles, alcoxy, acide carboxylique, hydroxyle, amine ou cétone.

Parmi les radicaux hétérocyliques préférés, on peut citer les groupes furyle, pyrannyle, pyrrolyle, imidazolyle, pyrazolyle, pyridyle, thiényle.

De préférence encore, les groupes hétérocycliques sont des groupes condensés tels que des groupes benzofurannyle, chromènyle, xanthényle, indolyle, isoindolyle, quinolyle, isoquinolyle, chromannyle, isochromannyle, indolinyle, isoindolinyle, coumarinyle, isocoumarinyle, ces groupes pouvant être substitués, en particulier par un ou plusieurs groupes OH.

Les précurseurs de colorant préférés sont :

- les flavanols comme la catéchine et le gallate d'épicatéchine,
- les flavonols comme la quercétine,
- les anthocyanidines comme la péonidine,
- les anthocyanines, par exemple l'oenine,
- les hydroxybenzoates, par exemple l'acide gallique,
- les flavones comme la lutéoline,
- les iridoïdes comme l'oleuropéine,

ces produits pouvant être osylés (par exemple glucosylés) et /ou sous forme d'oligomères (procyanidines) ;

- les hydroxystilbènes, par exemple le tétrahydroxy-3,3',4,5'-stilbène, éventuellement osylés (par exemple glucosylés) ;
- la 3,4-dihydroxyphénylalanine et ses dérivés ;
- la 2,3-dihydroxyphenylalanine et ses dérivés ;
- la 4,5-dihydroxyphenylalanine et ses dérivés ;
- le 4,5-dihydroxyindole et ses dérivés ;
- le 5,6-dihydroxyindole et ses dérivés ;
- le 6,7-dihydroxyindole et ses dérivés ;
- le 2,3-dihydroxyindole et ses dérivés ;
- les dihydroxycinnnamates tels que l'acide caféique et l'acide chlorogénique ;
- les hydroxycoumarines ;
- les hydroxyisocoumarines ;
- les hydroxycoumarones;
- les hydroxyisocoumarones;
- les hydroxychalcones ;
- les hydroxychromones ;
- les anthocyanes ;
- les quinones ;
- les hydroxyxanthones ; et
- les mélanges de ceux-ci.

Lorsque les précurseurs de colorant présentent des formes D et L, les deux formes peuvent être utilisées dans les compositions selon l'invention.

En faisant varier la nature des différents précurseurs de colorant et leurs proportions dans la composition, on peut faire varier la couleur de la composition de coloration finale. On obtient ainsi une palette de couleurs.

Les polymères formés en particulier avec la catéchine, l'acide gallique et leurs dérivés (tannins) ont des propriétés antimicrobiennes par emprisonnement des microorganismes lors de la polymérisation. Ces tannins ont également des propriétés astringentes intéressantes pour la peau.

Les précurseurs de colorants peuvent être des extraits de plantes, fruits, agrumes, légumes et des mélanges de ces extraits, qui contiennent de nombreux polyphénols tels que définis précédemment.

Parmi les extraits de plantes, on peut citer les extraits de rose, le sorgho et de thé.

Parmi les extraits de fruits, on peut citer les extraits de pomme, de raisin (en particulier de pépins de raisin), de cacao (fèves et/ou cabosses) et de banane.

Parmi les extraits de légumes, on peut citer l'extrait de pomme de terre.

On peut également utiliser des mélanges d'extraits de plantes et/ou de fruits tels que des mélanges d'extraits de pomme et de thé et des mélanges d'extraits de raisin et de pomme.

Suivant les parties de fruits utilisés, par exemple pulpe ou pépins de raisin, la coloration obtenue est différente.

La quantité de précurseur de colorant dans la composition finale doit être suffisante pour obtenir une coloration visible. Cette quantité peut varier dans de larges mesures en fonction de la nature du précurseur et de l'intensité voulue pour la coloration.

En général, on obtiendra une coloration convenable lorsque la quantité de précurseur de colorant est telle que la teneur en précurseur de colorant dans le composant (A) final est d'au moins 10 micromoles par millilitre de composant (A).

Le milieu physiologiquement acceptable du composant (A) tinctorial ou des composants ($A_1$) et ($A_2$) est un milieu solide ou liquide ne nuisant pas à la propriété de coloration des précurseurs ni à l'effet catalytique du système catalytique. Il est de préférence un milieu solubilisant du précurseur de colorant et à propriété bactériostatique.

Parmi les solvants des précurseurs convenant pour la formulation des compositions selon l'invention, on peut citer l'eau, les alcools, les solvants polaires et leurs mélanges.

Les alcools sont de préférence des alcanols inférieurs ($C_1$-$C_6$) tels que l'éthanol et l'isopropanol et les alcanediols tels que l'éthylèneglycol, le propylène glycol et le pentane diol.

Parmi les solvants polaires, on peut citer les éthers, les esters (en particulier les acétates), le diméthylsulfoxyde (DMSO), la N-méthylpyrrolidone (NMP), les cétones (en particulier l'acétone) et leurs mélanges.

Le milieu physiologiquement acceptable comprend de préférence de l'eau (en particulier distillée ou permutée) ou un mélange eau/alcool, en particulier eau/éthanol.

La quantité d'alcool dans le mélange eau/alcool peut représenter jusqu'à 80% en poids du mélange eau/alcool, de préférence 1 à 50% en poids et mieux 5 à 20% en poids.

De préférence, le composant (A) selon l'invention est exempte d'agent de chélation des sels de Mn (II) et/ou Zn (II) utilisés, car ces agents tendent à inhiber l'oxydation des précurseurs de colorant.

De préférence, le composant (A) selon l'invention ne contient pas d'enzyme propigmentante.

Les solvants sont, de préférence, présents dans des proportions de préférence comprises entre 1 et 40 % en poids environ par rapport au poids total de la composition (A) tinctoriale, et encore plus préférentiellement entre 5 et 30 % en poids environ.

Lorsqu'il est destiné à la coloration des cheveux, le composant tinctorial (A) sous forme unique ou les deux composants ($A_1$) et ($A_2$) conditionnés séparément peuvent se présenter sous des formes diverses, telles que sous forme de lotion, de crème, de gel, ou sous toute autre forme appropriée pour réaliser une teinture des fibres kératiniques, et notamment des cheveux humains. Il peut également renfermer divers adjuvants utilisés classiquement dans les compositions pour la teinture des cheveux, tels que des agents tensio-actifs anioniques, cationiques, non-ioniques, amphotères, zwitterioniques ou leurs mélanges, des polymères anioniques, cationiques, non-ioniques, amphotères, zwitterioniques ou leurs mélanges, des agents épaississants minéraux ou organiques, et en particulier les épaississants associatifs polymères anioniques, cationiques, non ioniques et amphotères, des agents antioxydants, des agents de pénétration, des parfums, des tampons, des agents dispersants, des agents de conditionnement tels que par exemple des silicones volatiles ou non volatiles, modifiées ou non modifiées, des agents filmogènes, des céramides, des agents opacifiants, des propulseurs.

Lorsqu'il est destiné à la coloration de la peau, le composant tinctorial (A) sous forme unique ou les deux composants ($A_1$) et ($A_2$) conditionnés séparément peuvent se présenter sous forme de crème, d'un lait, ou sous la forme d'un gel ou d'un gel crème, d'une lotion, d'une poudre, d'un solide ou sous toute autre forme appropriée pour réaliser une coloration de la peau. Il peut également renfermer divers adjuvants utilisés classiquement dans les compositions pour la coloration de la peau, tels que les corps gras, les solvants organiques, des agents tensio-actifs anioniques, cationiques, non-ioniques, amphotères, zwitterioniques ou leurs mélanges, des polymères anioniques, cationiques, non-ioniques, amphotères, zwitterioniques ou leurs mélanges, des agents épaississants minéraux ou organiques, et en particulier les épaississants associatifs polymères anioniques, cationiques, non ioniques et amphotères, les adoucissants, les antioxydants, les agents anti radicaux libres, les opacifiants, les émollients, les silicones, les agents anti-mousse, les agents hydratants, les vitamines, les agents répulsifs contre les insectes, les parfums, les tensioactifs, les anti-inflammatoires, les antagonistes de substance P, les charges, les propulseurs, les colorants, les filtres solaires organiques ou minéraux.

Le composant (A) sous forme unique ou les deux composants ($A_1$) et ($A_2$), indépendamment l'un de l'autre, peuvent être conditionnés sous différentes formes telles que par exemple sous forme de tube métallique hermétique à l'air, de sachet, de lingette scellée, d'ampoule, d'aérosol, de spray ou de bloc solide ou tout autre forme de conditionnement adaptée à la coloration du support kératinique choisi.

Selon une première réalisation, le composant (A) selon l'invention peut être conditionné dans un dispositif à un seul compartiment dans lequel se trouvent le ou les précurseurs de colorant et le système catalytique.

Ce dispositif peut être par exemple un tube métallique hermétique à l'air ; un sachet ; une lingette scellée ; une ampoule ; un aérosol contenant un ou plusieurs gaz propulseurs inertes classiques choisis parmi l'azote, les hydrocarbures saturés comme le butane, le propane, l'isopropane ou les hydrocarbures fluorés comme par exemple le Fréon® ; un spray muni d'une pompe sans reprise d'air ; un bloc solide comme un galet pour le bain.

Dans une seconde réalisation, le composant (A) selon l'invention peut être conditionné sous forme d'un kit comportant deux conteneurs distincts contenant respectivement les composants ($A_1$) et ($A_2$) tels que définis précédemment , les composants ($A_1$) et ($A_2$) étant mélangés ou appliqués successivement au moment de l'emploi.

Chacun des deux conteneurs, indépendamment l'un de l'autre peut être conditionné par exemple sous forme de tube hermétique à l'air, de sachet, de lingette scellée, d'ampoule, d'aérosol, de spray muni d'une pompe sans reprise d'air ou de bloc solide comme un galet pour le bain ou tout autre forme de conditionnement adaptée à la coloration du support kératinique choisi.

On peut prévoir également un dispositif aérosol à deux compartiments contenant respectivement les composants ($A_1$) et ($A_2$) et avec lesquels un orifice de distribution peut être mis en communication de manière sélective ; les composants ($A_1$) et ($A_2$) selon la configuration du dispositif pouvant être distribués simultanément ou successivement au moment de l'emploi.

On peut prévoir aussi un système à deux compartiments équipés chacun d'une pompe sans reprise d'air, le premier compartiment contenant le composant ($A_1$) avec le ou les précurseurs de colorant et un des catalyseurs (1) ou (2) tels que définis ci-dessus, et l'autre compartiment contenant le composant ($A_2$) avec l'autre des catalyseurs (1) ou (2) ; les composants ($A_1$) et ($A_2$) selon la configuration du dispositif pouvant être distribués simultanément ou successivement au moment de l'emploi.

Dans une troisième réalisation, le composant (A) unique ou à deux composants ($A_1$) et ($A_2$) selon l'invention peut se présenter sous forme d'un ou deux blocs solides délitables dans l'eau comme des galets pour le bain. Lesdits blocs solides peuvent être effervescents.

Dans l'agent de teinture conforme à l'invention, le composant (B) de fixation de la couleur comprend une composition aqueuse contenant au moins un acide minéral ou organique.

Le pH du composant (B) varie en général de 1 à 6 et plus préférentiellement de 2 à 5.

Parmi les acides minéraux utilisables selon l'invention, on peut citer par exemple l'acide chlorhydrique (HCl), l'acide phosphorique ($H_3PO_4$) ou leurs mélanges.

Le composé (B) peut être constitué d'une eau naturellement acide telle que par exemple une eau déminéralisée.

Parmi les acides organiques utilisables selon l'invention, on peut citer l'acide acétique, les $\alpha$-hydroxy-acides, les $\beta$-hydroxy-acides, les $\alpha$- et $\beta$-cétoacides et leurs mélanges.

Les hydroxy-acides sont choisis notamment parmi les acides glycolique, lactique, malique, tartrique, citrique, mandélique, salicylique ainsi que leurs dérivés alkylés comme l'acide n-octanoyl-5-salicylique, l'acide n-dodécanoyl-5-salicylique, l'acide 2-hydroxy-3-méthylbenzoïque, ou encore leurs dérivés alcoxylés comme l'acide 2-hydroxy-3-méthoxybenzoïque ou leurs mélanges. On utilisera préférentiellement selon l'invention l'acide lactique, l'acide glycolique ou l'acide citrique et leurs mélanges.

Le composant (B) de fixation de la couleur peut être conditionné sous différentes formes telles que notamment un flacon, un pot un tube, des sachets, des lingettes , un aérosol, un spray, un bâton solide ou tout autre forme de conditionnement adaptée à la coloration du support kératinique choisi.

Selon la présente invention, l'agent le composant (C) de ravivage de la couleur comprend une composition aqueuse contenant au moins une base minérale et/ou une base organique.

Le pH du composant (B) varie en général de 7 à 12 et plus préférentiellement de 8 à 10.

Parmi les bases minérales utilisables selon l'invention, on peut citer par exemple les sels de métal alcalin ou de métal alcalinoterreux comme la soude, la potasse ou l'ammoniaque ; les hydrogénocarbonates alcalins et alcalinoterreux tels que les hydrogénocarbonates de Na, K, Mg, Ca et plus particulièrement l'hydrogénocarbonate de Na ; ainsi que leurs mélanges. Le composant (C) peut être également constituée d'une eau minérale naturellement alcaline telle que l'Eau de Vichy, l'Eau de La Roche Posay.

Parmi les bases organiques, on peut citer par exemple les alcanolamines telle que la triéthanolamine.

Le composant (C) de ravivage de la couleur peut être conditionné sous différentes formes telles qu'un flacon, un pot, un tube, des sachets, des lingettes, un aérosol, un spray, un bâton solide ou tout autre forme de conditionnement adaptée à la coloration du support kératinique choisi.

Pour révéler la coloration des compositions suivant l'invention, il suffit de mettre la composition contenant au moins un précurseur de colorant et une quantité efficace du système catalytique selon l'invention, en présence d'un milieu oxydant tel qu'un milieu contenant de l'oxygène (par exemple l'oxygène de l'air).

Les compositions selon l'invention sont utiles pour la coloration de la peau, du cuir chevelu, des ongles ou des fibres kératiniques humaines telles que les cheveux, les cils, les sourcils et les poils. Différents procédés d'application des compositions selon l'invention peuvent être utilisés.

Selon un premier procédé de coloration, on applique dans un premier temps sur le support kératinique, en présence d'oxygène par exemple l'oxygène de l'air, un composant (A) unique comprenant au moins un précurseur de colorant et le système catalytique comprenant le catalyseur (1) et le catalyseur (2) tels que définis précédemment. Lorsque la teinte désirée est obtenue, on applique sur le support kératinique un composant (B) acide tel que défini précédemment pour fixer la couleur. Si l'on souhaite augmenter l'intensité de la couleur ou modifier la teinte , on applique ensuite sur le support kératinique un composant (C) alcalin tel que défini précédemment ; la nouvelle couleur obtenue pouvant à nouveau être fixée par une nouvelle application de la composition (B).

Selon un deuxième procédé de coloration, on applique dans un premier temps sur le support kératinique un composant ($A_1$) comprenant au moins un précurseur de colorant et un des catalyseurs (1) et (2) puis dans un deuxième temps on révèle la couleur en présence d'oxygène par exemple l'oxygène de l'air, en appliquant un composant ($A_2$) comprenant l'autre des catalyseurs (1) et (2). Lorsque la teinte désirée est obtenue, on applique sur le support kératinique un composant (B) acide tel que défini précédemment pour fixer la couleur. Si l'on souhaite augmenter l'intensité de la couleur ou modifier la teinte, on applique sur le support kératinique un composant (C) alcalin tel que défini précédemment ; la nouvelle couleur obtenue pouvant à nouveau être fixée par une nouvelle application de la composition (B).

Selon un troisième procédé de coloration, on applique dans un premier temps sur le support kératinique, en présence d'oxygène par exemple l'oxygène de l'air, un composant (A) unique comprenant au moins un précurseur de

colorant et le système catalytique comprenant le catalyseur (1) et le catalyseur (2) tels que définis précédemment. Si l'on souhaite augmenter l'intensité de la couleur ou modifier la teinte, on applique ensuite sur le support kératinique un composant (C) alcalin tel que défini précédemment ; la nouvelle couleur obtenue pouvant être fixée par application de la composition (B) tel que défini précédemment.

Selon un quatrième procédé de coloration, on applique dans un premier temps sur le support kératinique, , un composant ($A_1$) comprenant au moins un précurseur de colorant et un des catalyseur (1) et (2) puis dans un deuxième temps on révèle la couleur en présence d'oxygène par exemple l'oxygène de l'air, en appliquant un composant ($A_2$) comprenant l'autre des catalyseurs (1) et (2). Si l'on souhaite augmenter l'intensité de la couleur ou modifier la teinte, on applique sur le support kératinique un composant (C) alcalin tel que défini précédemment ; la nouvelle couleur obtenue pouvant être fixée par application de la composition (B) tel que défini précédemment.

Les agents de teinture de l'invention, en fonction du choix des précurseurs de colorant, peuvent être utilisés dans de nombreuses applications cosmétiques. Ils peuvent être utilisés pour la teinture des cheveux.

Dans le cadre de la cosmétique spécifique à la peau, les agents de teinture de l'invention peuvent constituer des compositions pour le bronzage et/ou le brunissement artificiel de la peau, et /ou la bonne mine.

Dans le cadre de la cosmétique spécifique à la peau, les agents de teinture de l'invention peuvent constituer des compositions pour le maquillage de la peau notamment pour réaliser des tatouages par l'intermédiaire de pochoirs en modulant les couleurs. Ils peuvent également être utilisés pour moduler la couleur suivant les zones de relief du visage. Ils peuvent être appliqués sur le visage ou les mains pour masquer des défauts de pigmentation tels que le vitiligo, le masque de grossesse, ainsi que des imperfections de la peau comme des cicatrices, les tâches de vieillissement, le chloasma et la couperose.

Les agents de teinture de l'invention peuvent constituer des compositions pour le maquillage des ongles, des cils et des sourcils.

Les agents de teinture de l'invention peuvent constituer des compositions pour la teinture des fibres textiles kératiniques. Les agents de teinture de l'invention peuvent être utilisés également pour la coloration alimentaire.

Les exemples suivants illustrent la présente invention. Dans les exemples, sauf indication contraire, tous les pourcentages et parties sont exprimés en poids.

**Exemple 1**

**Composant A à deux composants $A_1$ et $A_2$ suivants**

| Composant $A_1$ : crème blanche | | | |
|---|---|---|---|
| Phase $a_1$ : | Glycéryle stéarate (et) PEG-100 stéarate | | 2,5 % |
| | Polysorbate 60 | | 2,5 % |
| | Alcool cétylique | | 1 % |
| | Alcool stéarylique | | 1 % |
| | Paraffine | | 5 % |
| | Conservateur | | 0,1 % |
| Phase $b_1$ : | Conservateur | | 0,2 % |
| | Carbomer | | 0,3 % |
| | Base | | 0,2 % |
| | Catéchine | | 0.2% |
| | Propyl gallate | | 2% |
| | $MnCl_2$ | | 0.0002% |
| | Eau | qsp | 100 % |

| Composant $A_2$ : crème blanche | | |
|---|---|---|
| Phase $a_2$ : | Glycéryle stéarate (et) PEG-100 stéarate | 2,5 % |
| | Polysorbate 60 | 2,5 % |
| | Alcool cétylique | 1 % |
| | Alcool stéarylique | 1 % |

(suite)

| Composant $A_2$ : crème blanche | | | |
|---|---|---|---|
| | Paraffine | | 5 % |
| | Conservateur | | 0,1 % |
| Phase $b_2$ : | Conservateur | | 0,2 % |
| | Carbomer | | 0,3 % |
| | Bicarbonate de sodium | | 1% |
| | Base | | 0,2 % |
| | Eau | qsp | 100 % |

***Mode opératoire :***

Chaque phase $a_1$ ou $a_2$ est préparée par homogénéisation des différents constituants à 75°C. Chaque phase $b_1$ ou $b_2$ est ajoutée respectivement à la phase $a_1$ ou $a_2$ à une température de 75°C. Le mélange est refroidit à 25°C

Chacune de ces préparations est introduite dans un des compartiments d'un flacon pompe à deux compartiments et se mélangera à l'autre en sortie de pompe ce qui amènera à produire une crème teintée.

| Composant B : Gel aqueux acide | | | |
|---|---|---|---|
| Phase 1 | Acrylates/$C_{10}$-$C_{30}$ alkyl acrylate crosspolymer | | 0,25 % |
| | Base | | 0,15 % |
| Phase 2 | Xanthane | | 0,3 % |
| | Glycérol | | 3 % |
| | Propylène glycol | | 3 % |
| | PEG-8 | | 3 % |
| | Acide lactique | | 0.5 % |
| | Conservateur | | 0,1 % |
| | Eau | qsp | 100 % |

**Mode opératoire**

**Phase 1 :** On disperse l'acide carboxylique dans l'eau puis on le neutralise par la base

**Phase 2 :** La phase est préparée par homogénéisation des différents constituants. Le mélange phase 1 et 2 est ensuite opéré.Le pH obtenu est inférieur à 5

| Composé C : Solution aqueuse basique | | |
|---|---|---|
| Glycérol | | 3 % |
| PEG-8 | | 3 % |
| Triéthanolamine | | 0.9% |
| Conservateur | | 0,1 % |
| Eau de Vichy (Eau de Lucas) | | 5% |
| Eau | qsp | 100 % |

La triéthanolamine peut-être avantageusement remplacée par la soude.

On applique au moyen du flacon pompe le composant (A) sur la peau. On obtient au bout d'environ de 5 minutes une couleur homogène donnant à l'utilisateur un teint halé. Puis on fixe la couleur ainsi obtenue en appliquant sur la zone colorée de la peau le gel acide (B). Au bout de quelques heures, selon le souhait de l'utilisateur, on peut raviver la couleur (rétablir la première teinte obtenue en appliquant (A) puis (B)) en appliquant la solution (C) suivie éventuellement de l'application du produit (B) pour fixer à nouveau la couleur.

## Revendications

1. Procédé de coloration des matières kératiniques, **caractérisé qu'**on applique

   (i) dans un premier temps sur le support kératinique, en présence d'oxygène par exemple l'oxygène de l'air, un composant (A) unique comprenant dans un milieu physiologiquement acceptable, au moins un précurseur de colorant choisi parmi les composés comportant au moins un cycle aromatique ayant au moins deux groupes hydroxyles portés par deux atomes de carbone consécutifs du cycle aromatique et au moins un système catalytique comprenant un premier catalyseur (1) choisi parmi les sels et oxydes de Mn (II) et/ou de Zn (II) et leurs mélanges et un second catalyseur (2) choisi parmi les hydrogénocarbonates alcalins, les hydrogénocarbonates alcalino-terreux et leurs mélanges ;
   (ii) lorsque la teinte désirée est obtenue, on applique sur le support kératinique un composant (B) comprenant une composition acide pour fixer la couleur ;
   (iii) on applique ensuite sur le support kératinique un composant (C) comprenant une composition alcaline afin d'augmenter l'intensité de la couleur ou modifier la teinte,
   (iv) la nouvelle couleur obtenue pouvant à nouveau être fixée par une nouvelle application de la composition (B).

2. Procédé selon la revendication 1, **caractérisé par le fait qu'**on applique

   (i) dans un premier temps sur le support kératinique un composant ($A_1$) comprenant au moins un précurseur de colorant et un des catalyseurs (1) ou (2) tels que définis dans la revendication 1 puis dans un deuxième temps on révèle la couleur en présence d'oxygène par exemple l'oxygène de l'air, en appliquant un composant ($A_2$) comprenant l'autre des catalyseurs (1) ou (2) ;
   (ii) lorsque la teinte désirée est obtenue, on applique sur le support kératinique un composant (B) comprenant une composition acide pour fixer la couleur ;
   (iii) si l'on souhaite augmenter l'intensité de la couleur ou modifier la teinte , on applique ensuite sur le support kératinique un composant (C) comprenant une composition alcaline;
   (iv) la nouvelle couleur obtenue pouvant à nouveau être fixée par une nouvelle application de la composition (B).

3. Procédé de coloration des matières kératiniques, **caractérisé qu'**on applique

   (i) dans un premier temps sur le support kératinique, en présence d'oxygène par exemple l'oxygène de l'air, un composant (A) unique tel que défini dans la revendication 1 ;
   (ii) on applique ensuite sur le support kératinique un composant (C) comprenant une composition alcaline afin d'augmenter l'intensité de la couleur ou modifier la teinte ;
   (iii) la nouvelle couleur obtenue pouvant être fixée par application d'un composant (B) comprenant une composition acide.

4. Procédé selon la revendication 3, **caractérisé par le fait qu'**on applique :

   (i) dans un premier temps sur le support kératinique un composant ($A_1$) comprenant au moins un précurseur de colorant et un des catalyseur (1) ou (2) tels que définis dans la revendication 1 puis dans un deuxième temps on révèle la couleur en présence d'oxygène par exemple l'oxygène de l'air, en appliquant un composant ($A_2$) comprenant l'autre des catalyseurs (1) ou (2) ;
   (ii) on applique sur le support kératinique un composant (C) comprenant une composition alcaline ;
   (iii) la nouvelle couleur obtenue pouvant être fixée par application d'un composant (B comprenant une composition acide.

5. Procédé selon l'une quelconque des revendications 1 à 4, où dans le composant (A) ou dans les composants ($A_1$) et ($A_2$), les proportions du premier catalyseur (1) et du second catalyseur (2) sont de choisies de telle sorte que :

$$\frac{[Mn(II)]}{[HCO_3]} \leq 1 \text{ avec } [\mathrm{Mn(II)}] \neq 0$$

$$\frac{[Zn(II)]}{[HC0_3]} \leq 1 \text{ avec } [Zn(II)] \neq 0$$

$$\frac{[Mn(II) + Zn(II)]}{[HCO_3]} \leq 1 \text{ avec } [Mn(II)] \text{ et } [Zn(II)] \neq 0$$

où [Mn(II)], [Zn(II)] et [$HCO_3$] représentent respectivement les concentrations molaires en Mn(II), Zn(II) et $HCO_3$ dans la composition.

6. Procédé selon la revendication 5, **caractérisé en ce que** le rapport $\frac{[Mn(II)]}{[HCO_3]}$ varie de $10^{-5}$ à $10^{-1}$, de préférence de $10^{-3}$ à $10^{-2}$ et mieux encore de l'ordre de $5.10^{-3}$.

7. Procédé selon la revendications 5 ou 6, **caractérisé en ce que** le rapport $\frac{[Zn(II)]}{[HCO_3]}$ varie de $10^{-4}$ à < 1, de préférence de $10^{-3}$ à < 1, et mieux encore de l'ordre de $5.10^{-1}$.

8. Procédé selon l'une quelconque des revendications 5 à 7, **caractérisé en ce que** le rapport $\frac{[Mn(II) + Zn(II)]}{[HCO_3]}$ varie de $10^{-5}$ à $10^{-1}$, de préférence $10^{-3}$ à $10^{-2}$.

9. Procédé selon l'une quelconque des revendications 1 à 8, **caractérisé en ce que** les sels de Mn(II) et de Zn(II) du composant (A) ou des composants ($A_1$) et ($A_2$) sont choisis parmi les chlorure, fluorure, iodure, sulfate, phosphate, nitrate, perchlorate, les sels d'acides carboxyliques et leurs mélanges.

10. Procédé selon la revendication 9, **caractérisé en ce que** le sel de Mn(II) et/ou de Zn(II) est le chlorure.

11. Procédé selon la revendication 9, **caractérisée en ce que** les sels d'acides carboxyliques sont des sels d'acides carboxyliques hydroxylés.

12. Procédé selon la revendication 11, **caractérisé en ce que** le sel d'acide carboxylique hydroxylé est le gluconate.

13. Procédé selon l'une quelconque des revendications 1 à 12, **caractérisée en ce que** l'hydrogénocarbonate est choisi parmi l'hydrogénocarbonate de sodium, l'hydrogénocarbonate de potassium, l'hydrogénocarbonate de magnésium, l'hydrogénocarbonate de calcium et leurs mélanges.

14. Procédé selon l'une quelconque des revendications 1 à 13, **caractérisée en ce que** le cycle aromatique comportant au moins deux groupes hydroxyle sur deux atomes de carbone consécutifs du précurseur de colorant est un cycle benzénique ou un cycle aromatique condensé.

15. Procédé selon la revendication 14, **caractérisé en ce que** le précurseur de colorant est un composé de formule :

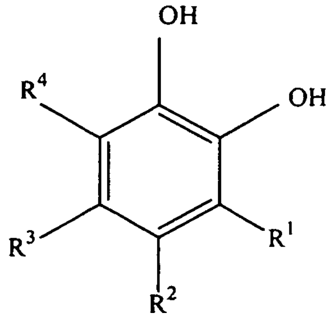

(I)

dans laquelle les substituts $R^1$ à $R^4$, identiques ou différents, représentent un atome d'hydrogène, un halogène, un radical hydroxyle, carboxyle, carboxylate d'alkyle, amino éventuellement substitué, alkyle linéaire ou ramifié éventuellement substitué, alcényle linéaire ou ramifié éventuellement substitué, cycloalkyle éventuellement substitué, alcoxy, alcoxyalkyle, alcoxyaryle, le groupe aryle pouvait être éventuellement substitué, aryle, aryle substitué, un radical hétérocyclique éventuellement substitué, un radical contenant éventuellement un ou plusieurs atomes de silicium, où deux des substituants $R^1$ à $R^4$ peuvent former conjointement un cycle saturé ou insaturé contenant éventuellement un ou plusieurs hétéroatomes et éventuellement condensé avec un ou plusieurs cycles saturés ou insaturés contenant éventuellement un ou plusieurs hétéroatomes.

**16.** Procédé selon l'une quelconque des revendications 1 à 13, **caractérisée en ce que** le précurseur de colorant est choisi parmi les flavanols, les flavonols, les anthocyaninidines, les anthocyanines, les hydroxybenzoates, les flavones, les iridoïdes, ces composés pouvant être éventuellement osylés et/ou sous forme d'oligomères, les hydroxystilbènes éventuellement osylés, la 3,4-dihydroxyphénylalanine et ses dérivés, la 2,3-dihydroxyphénylalanine et ses dérivés, la 4,5-dihydroxyphénylalanine et ses dérivés, le 4,5-dihydroxyindole et ses dérivés, le 5,6-dihydroxyindole et ses dérivés, le 6,7-dihydroxyindole et ses dérivés, le 2,3-dihydroxyindole et ses dérivés, les dihydroxycinnamates, les hydroxycoumarines, les hydroxyisocoumarines, les hydroxycoumarones, les hydroxyisocoumarones, les hydroxychalcones, les hydroxychromones, les anthocyanes, les quinones, les hydroxyxantones, et les mélanges de deux ou plus des composés précédents.

**17.** Procédé selon l'une quelconque des revendications 1 à 16, **caractérisé en ce que** le précurseur de colorant est choisi parmi les extraits de plantes, de fruits, d'agrumes, de légumes et leurs mélanges.

**18.** Procédé selon la revendication 17, **caractérisé en ce que** le précurseur de colorant est choisi parmi les extraits de thé, de raisin, de pomme, de cacao, de sorgho, de banane, de pomme de terre et leurs mélanges.

**19.** Procédé selon l'une quelconque des revendications 1 à 18, **caractérisé en ce que** le précurseur de colorant est présent dans le composant (A) finale en une quantité d'au moins 10 micromoles par millilitre de composant (A).

**20.** Procédé selon l'une quelconque des revendications 1 à 18, **caractérisée en ce que** le milieu physiologiquement acceptable du composant (A) ou des composants ($A_1$) et ($A_2$) est un milieu solubilisant du précurseur de colorant, de préférence à propriété bactériostatique.

**21.** Procédé selon l'une quelconque des revendications 1 à 20, **caractérisé en ce que** le milieu physiologiquement acceptable du composant (A) ou des composants ($A_1$) et ($A_2$) comprend un solvant ou un mélange de solvants du précurseur de colorant.

**22.** Procédé selon la revendication 21, **caractérisé en ce que** le solvant est choisi parmi l'eau, les alcools, les éthers, le diméthylsulfoxyde, la N-méthylpyrrolidone, les acétones et leurs mélanges.

**23.** Procédé selon la revendication 22, **caractérisé en ce que** l'alcool est un alcanol ou un alcanediol.

**24.** Procédé selon la revendication 22, **caractérisé en ce que** le solvant est un mélange eau/alcool.

**25.** Procédé selon la revendication 24, **caractérisé en ce que** l'alcool représente jusqu'à 80% en poids du mélange, de préférence 1 à 50% en poids et mieux de 5 à 20% en poids.

**26.** Procédé selon l'une quelconque des revendications 1 à 25, **caractérisée en ce que** le composant (A) est exempte de tout agent de chélation du sel de Mn(II) et/ou Zn(II).

**27.** Procédé selon l'une quelconque des revendications 1 à 26, **caractérisée en ce que** le composant (A) ne contient pas d'enzyme propigmentante.

**28.** Procédé selon l'une quelconque des revendications 1 à 27, où le composant (A) sous forme unique ou les composants ($A_1$) et ($A_2$) se présentent sous forme de crème, de lait, de gel, de gel crème, de lotion, de poudre, de bloc solide.

**29.** Procédé selon l'une quelconque des revendications 1 à 28, où le composant (A) est conditionné dans un dispositif à un seul compartiment dans lequel se trouvent le ou les précurseurs de colorant et le système catalytique.

**30.** Procédé selon la revendication 29 où le dispositif est choisi dans le groupe constitué par un tube métallique hermétique à l'air, une ampoule, un sachet, une lingette scellée, un aérosol contenant un ou un plusieurs gaz propulseurs inertes classiques, un dispositif à pompe sans reprise d'air ou un bloc solide.

**31.** Procédé selon l'une quelconque des revendications 1 à 30, où les deux composants ($A_1$) et ($A_2$) sont est conditionnés sous forme d'un kit comportant deux conteneurs distincts ; le premier conteneur contenant le composant ($A_1$) comprenant le ou les précurseurs de colorant et un des catalyseurs (1) ou (2) tels que définis dans les revendications précédentes, l'autre conteneur contenant le composant ($A_2$) comprenant l'autre des catalyseurs (1) ou (2), les composants ($A_1$) et ($A_2$) étant mélangés ou appliqués successivement au moment de l'emploi.

**32.** Procédé selon la revendication 31, où chaque conteneur, indépendamment l'un de l'autre, est conditionné dans un dispositif choisi dans le groupe constitué par un tube métallique hermétique à l'air, une ampoule, un sachet, une lingette scellée, un aérosol contenant un ou un plusieurs gaz propulseurs inertes classiques, un dispositif à pompe sans reprise d'air ou un bloc solide.

**33.** Procédé selon la revendication 31, où le dispositif est un aérosol à deux compartiments contenant respectivement les composants ($A_1$) et ($A_2$) et avec lesquels un orifice de distribution peut être mis en communication de manière sélective ; les composants ($A_1$) et ($A_2$) selon la configuration du dispositif pouvant être distribués simultanément ou successivement au moment de l'emploi.

**34.** Procédé selon la revendication 31, où le dispositif est un système à deux compartiments équipés chacun d'une pompe sans reprise d'air, le premier compartiment contenant le composant ($A_1$), et l'autre compartiment contenant le composant ($A_2$) ; les composants ($A_1$) et ($A_2$) selon la configuration du dispositif pouvant être distribués simultanément ou successivement au moment de l'emploi.

**35.** Procédé selon l'une quelconque des revendications 1 à 34 où le composant (A) ou les composants ($A_1$) et ($A_2$) se présentent sous forme de blocs solides délitables dans l'eau.

**36.** Procédé selon l'une quelconque des revendications 1 à 35 où le composant (B) comprend une composition aqueuse contenant au moins un acide minéral ou organique.

**37.** Procédé selon la revendication 36 34 où le pH du composant (B) varie en général de 1 à 6 et plus préférentiellement de 2 à 5.

**38.** Procédé selon la revendication 36 ou 37, où l'acide minéral est choisi parmi l'acide chlorhydrique (HCl), l'acide phosphorique ($H_3PO_4$) ou leurs mélanges.

**39.** Procédé selon la revendication 36 ou 37, où le composant (B) est constituée d'une eau naturellement acide.

**40.** Procédé selon la revendication 36 ou 37, où l'acide organique est choisi parmi l'acide acétique, les $\alpha$-hydroxy-acides, les $\beta$-hydroxy-acides, les $\alpha$- et $\beta$-cétoacides ou leurs mélanges.

**41.** Procédé selon la revendication 36 ou 37, où l'acide organique est choisi parmi les acides glycolique, lactique, malique, tartrique, citrique, mandélique, salicylique ainsi que leurs dérivés alkylés ou alcoxylés et leurs mélanges.

**42.** Procédé selon la revendication 41, où l'acide organique est choisi parmi l'acide lactique, l'acide glycolique, l'acide

citrique et leurs mélanges.

**43.** Procédé selon l'une quelconque des revendications 1 à 42, où le composant (B) est conditionné sous forme de flacon, de pot, de tube, de sachet, de lingette, d' aérosol, de spray, de bâton solide.

**44.** Procédé selon l'une quelconque des revendications 1 à 43, où le composant (C) comprend une composition aqueuse contenant au moins une base minérale et/ou une base organique.

**45.** Procédé selon la revendication 44, où le pH du composant (C) varie en général de 7 à 12 et plus préférentiellement de8à10.

**46.** Procédé selon la revendication 44, où la base minérale est choisi parmi les sels de métal alcalin ou de métal alcalino-terreux ; les hydrogénocarbonates alcalins et alcalino-terreux et leurs mélanges

**47.** Procédé selon la revendication 44 ou 45, où le composant (C) est une eau minérale naturellement alcaline.

**48.** Procédé selon la revendication 44, où la base organique est choisie parmi les alcanolamines.

**49.** Procédé selon l'une quelconque des revendications 1 à 48, où le composant (C) est conditionné sous forme de flacon, de pot, de tube, de sachet, de lingette, d' aérosol, de spray, de bâton solide.

## Claims

**1.** Process for colouring keratin materials, **characterized in that**:

(i) in a first stage, a single component (A) comprising, in a physiologically acceptable medium, at least one dye precursor chosen from compounds comprising at least one aromatic ring containing at least two hydroxyl groups borne by two consecutive carbon atoms of the aromatic ring, and at least one catalytic system comprising a first catalyst (1) chosen from Mn(II) and/or Zn(II) salts and oxides, and mixtures thereof, and a second catalyst (2) chosen from alkali metal hydrogen carbonates and alkaline-earth metal hydrogen carbonates, and mixtures thereof, is applied to the keratin support, in the presence of oxygen, for example atmospheric oxygen;
(ii) when the desired shade is obtained, a component (B) comprising an acidic composition is applied to the keratin support to fix the colour;
(iii) a component (C) comprising an alkaline composition is then applied to the keratin support in order to increase the intensity of the colour or to modify the shade;
(iv) the new colour obtained possibly being fixed again by means of a new application of composition (B).

**2.** Process according to Claim 1, **characterized in that**:

(i) in a first stage, a compound and ($A_1$) comprising at least one dye precursor and one of the catalysts (1) or (2) as defined in Claim 1 is applied to the keratin support, and then, in a second stage, the colour is developed in the presence of oxygen, for example atmospheric oxygen, by applying a component ($A_2$) comprising the other of the catalysts (1) or (2);
(ii) when the desired shade is obtained, a component (B) comprising an acidic composition is applied to the keratin support to fix the colour;
(iii) if it is desired to increase the intensity of the colour or to modify the shade, a component (C) comprising an alkaline composition is then applied to the keratin support;
(iv) the new colour obtained possibly being fixed again by means of a new application of composition (B).

**3.** Process for colouring keratin materials, **characterized in that**:

(i) in a first stage, a single component (A) as defined in Claim 1 is applied to the keratin support, in the presence of oxygen, for example atmospheric oxygen;
(ii) a component (C) comprising an alkaline composition is then applied to the keratin support in order to increase the intensity of the colour or to modify the shade;
(iii) the new colour obtained possibly being fixed by means of applying a component (B) comprising an acidic composition.

**4.** Process according to Claim 3, **characterized in that**:

(i) in a first stage, a component ($A_1$) comprising at least one dye precursor and one of the catalysts (1) or (2) as defined in Claim 1 is applied to the keratin support, and then, in a second stage, the colour is developed in the presence of oxygen, for example atmospheric oxygen, by applying a component ($A_2$) comprising the other of the catalysts (1) or (2);
(ii) a component (C) comprising an alkaline composition is applied to the keratin support;
(iii) the new colour obtained possibly being fixed by means of applying a component (B) comprising an acidic composition.

**5.** Process according to any one of Claims 1 to 4, in which, in the component (A) or in the components ($A_1$) and ($A_2$), the proportions of the first catalyst (1) and of the second catalyst (2) are chosen such that:

$$\frac{[Mn(II)]}{[HCO_3]} \leq 1 \text{ with } [Mn(II)] \neq 0$$

$$\frac{[Zn(II)]}{[HCO_3]} \leq 1 \text{ with } [Zn(II)] \neq 0$$

$$\frac{[Mn(II)+Zn(II)]}{[HCO_3]} \leq 1 \text{ with } [Mn(II)] \text{ and } [Zn(II)] \neq 0$$

in which [Mn(II)], [Zn(II)] and [$HCO_3$] represent, respectively, the molar concentrations of Mn(II), Zn(II) and $HCO_3$ in the composition.

**6.** Process according to Claim 5, **characterized in that** the ratio $\frac{[Mn(II)]}{[HCO_3]}$ ranges from $10^{-5}$ to $10^{-1}$, preferably from $10^{-3}$ to $10^{-2}$ and better still is about $5 \times 10^{-3}$.

**7.** Process according to Claim 5 or 6, **characterized in that** the ratio $\frac{[Zn(II)]}{[HCO_3]}$ ranges from $10^{-4}$ to < 1, preferably from $10^{-3}$ to < 1 and better still is about $5 \times 10^{-1}$.

**8.** Process according to any one of Claims 5 to 7, **characterized in that** the ratio $\frac{[Mn(II)+Zn(II)]}{[HCO_3]}$ ranges from $10^{-5}$ to $10^{-1}$ and preferably from $10^{-3}$ to $10^{-2}$.

**9.** Process according to any one of Claims 1 to 8, **characterized in that** the Mn(II) and Zn(II) salts of the component (A) or of the components ($A_1$) and ($A_2$) are chosen from the chloride, fluoride, iodide, sulfate, phosphate, nitrate and perchlorate, carboxylic acid salts, and mixtures thereof.

**10.** Process according to Claim 9, **characterized in that** the Mn(II) and/or Zn(II) salt is the chloride.

**11.** Process according to Claim 9, **characterized in that** the carboxylic acid salts are hydroxylated carboxylic acid salts.

**12.** Process according to Claim 11, **characterized in that** the hydroxylated carboxylic acid salt is the gluconate.

**13.** Process according to any one of Claims 1 to 12, **characterized in that** the hydrogen carbonate is chosen from

sodium hydrogen carbonate, potassium hydrogen carbonate, magnesium hydrogen carbonate and calcium hydrogen carbonate, and mixtures thereof.

**14.** Process according to any one of Claims 1 to 13, **characterized in that** the aromatic ring comprising at least two hydroxyl groups on two consecutive carbon atoms of the dye precursor is a benzene ring or a fused aromatic ring.

**15.** Process according to Claim 14, **characterized in that** the dye precursor is a compound of formula:

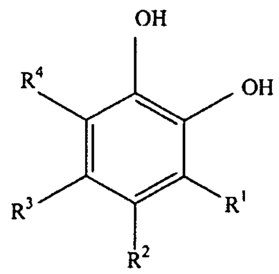

(I)

in which the substituents $R^1$ to $R^4$, which may be identical or different, represent a hydrogen atom, a halogen, a hydroxyl, carboxyl, alkylcarboxylate, optionally substituted amino, optionally substituted linear or branched alkyl, optionally substituted linear or branched alkenyl, optionally substituted cycloalkyl, alkoxy, alkoxyalkyl or alkoxyaryl radical, the aryl group possibly being optionally substituted, an aryl or substituted aryl radical, an optionally substituted heterocyclic radical, or a radical optionally containing one or more silicon atoms, in which two of the substituents $R^1$ to $R^4$ together form a saturated or unsaturated ring optionally containing one or more heteroatoms and optionally fused with one or more saturated or unsaturated rings optionally containing one or more heteroatoms.

**16.** Process according to any one of Claims 1 to 13, **characterized in that** the dye precursor is chosen from flavanols, flavonols, anthocyanidins, anthocyanins, hydroxybenzoates, flavones and iridoids, these compounds possibly being osylated and/or in the form of oligomers, hydroxystilbenes which are optionally osylated, 3,4-dihydroxyphenylalanine and its derivatives, 2,3-dihydroxyphenylalanine and its derivatives, 4,5-dihydroxyphenylalanine and its derivatives, 4,5-dihydroxyindole and its derivatives, 5,6-dihydroxyindole and its derivatives, 6,7-dihydroxyindole and its derivatives, 2,3-dihydroxyindole and its derivatives, dihydroxycinnamates, hydroxycoumarins, hydroxyisocoumarins, hydroxycoumarones, hydroxyisocoumarones, hydroxychalcones, hydroxychromones, anthocyans, quinones and hydroxyxanthones, and mixtures of two or more of the above compounds.

**17.** Process according to any one of Claims 1 to 16, **characterized in that** the dye precursor is chosen from extracts of plants, of fruits, of citrus plants and of vegetables, and mixtures thereof.

**18.** Process according to Claim 17, **characterized in that** the dye precursor is chosen from extracts of tea, of grape, of apple, of cocoa, of sorghum, of banana and of potato, and mixtures thereof.

**19.** Process according to any one of Claims 1 to 18, **characterized in that** the dye precursor is present in the final component (A) in an amount of at least 10 micromol per millilitre of component (A).

**20.** Process according to any one of Claims 1 to 18, **characterized in that** the physiologically acceptable medium for the component (A) or for the components ($A_1$) and ($A_2$) is a solubilizing medium for the dye precursor, preferably with bacteriostatic properties.

**21.** Process according to any one of Claims 1 to 20, **characterized in that** the physiologically acceptable medium for the component (A) or for the components ($A_1$) and ($A_2$) comprises a solvent or a mixture of solvents for the dye precursor.

**22.** Process according to Claim 21, **characterized in that** the solvent is chosen from water, alcohols, ethers, dimethyl sulfoxide, N-methylpyrrolidone and acetones, and mixtures thereof.

**23.** Process according to Claim 22, **characterized in that** the alcohol is an alkanol or an alkanediol.

**24.** Process according to Claim 22, **characterized in that** the solvent is a water/alcohol mixture.

**25.** Process according to Claim 24, **characterized in that** the alcohol represents up to 80% by weight of the mixture, preferably 1% to 50% by weight and better still from 5% to 20% by weight.

**26.** Process according to any one of Claims 1 to 25, **characterized in that** the component (A) is free of any agent for chelating the Mn(II) and/or Zn(II) salt.

**27.** Process according to any one of Claims 1 to 26, **characterized in that** the component (A) contains no propigmenting enzyme.

**28.** Process according to any one of Claims 1 to 27, in which the component (A) in the form of a single component or the components ($A_1$) and ($A_2$) are in the form of a cream, a milk, a gel, a cream-gel, a lotion, a powder or a solid block.

**29.** Process according to any one of Claims 1 to 28, in which the component (A) is packaged in a one-compartment device containing the dye precursor(s) and the catalytic system.

**30.** Process according to Claim 29, in which the device is chosen from the group consisting of an airtight metal tube, an ampule, a sachet, a sealed wipe, an aerosol containing one or more standard inert propellent gases, a pump device without air intake or a solid block.

**31.** Process according to any one of Claims 1 to 30, in which the two components ($A_1$) and ($A_2$) are packaged in the form of a kit comprising two separate containers; the first container containing the component ($A_1$) comprising the dye precursor(s) and one of the catalysts (1) or (2) as defined in the preceding claims, the other container containing the component ($A_2$) comprising the other catalyst (1) or (2), the components ($A_1$) and ($A_2$) being mixed together or applied successively at the time of use.

**32.** Process according to Claim 31, in which each container, independently of each other, is packaged in a device chosen from the group consisting of an airtight metal tube, an ampule, a sachet, a sealed wipe, an aerosol containing one or more standard inert propellent gases, a pump device without air intake or a solid block.

**33.** Process according to Claim 31, in which the device is a two-compartment aerosol containing, respectively, the components ($A_1$) and ($A_2$) and with which a distribution orifice may be selectively placed in communication; depending on the configuration of the device, the components ($A_1$) and ($A_2$) may be distributed simultaneously or successively at the time of use.

**34.** Process according to Claim 31, in which the device is a system containing two compartments each equipped with a pump without air intake, the first compartment containing the component ($A_1$), and the other compartment containing the component ($A_2$); depending on the configuration of the device, the components ($A_1$) and ($A_2$) may be distributed simultaneously or successively at the time of use.

**35.** Process according to any one of Claims 1 to 34, in which the component (A) or the components ($A_1$) and ($A_2$) are in the form of solid blocks that may be disintegrated in water.

**36.** Process according to any one of Claims 1 to 35, in which the component (B) comprises an aqueous composition containing at least one mineral or organic acid.

**37.** Process according to Claim 36, in which the pH of the component (B) generally ranges from 1 to 6 and more preferably from 2 to 5.

**38.** Process according to Claim 36 or 37, in which the mineral acid is chosen from hydrochloric acid (HCl) and phosphoric acid ($H_3PO_4$), or mixtures thereof.

**39.** Process according to Claim 36 or 37, in which the component (B) consists of a naturally acidic water.

**40.** Process according to Claim 36 or 37, in which the organic acid is chosen from acetic acid, $\alpha$-hydroxy acids, $\beta$-hydroxy acids and $\alpha$- and $\beta$-keto acids, or mixtures thereof.

**41.** Process according to Claim 36 or 37, in which the organic acid is chosen from glycolic acid, lactic acid, malic acid, tartaric acid, citric acid, mandelic acid and salicylic acid, and also alkyl or alkoxy derivatives thereof, and mixtures thereof.

**42.** Process according to Claim 41, in which the organic acid is chosen from lactic acid, glycolic acid and citric acid, and mixtures thereof.

**43.** Process according to any one of Claims 1 to 42, in which the component (B) is packaged in the form of a bottle, a jar, a tube, a sachet, a wipe, an aerosol, a spray or a solid stick.

**44.** Process according to any one of Claims 1 to 43, in which the component (C) comprises an aqueous composition containing at least one mineral base and/or one organic base.

**45.** Process according to Claim 44, in which the pH of the component (C) generally ranges from 7 to 12 and more preferably from 8 to 10.

**46.** Process according to Claim 44, in which the mineral base is chosen from alkali metal and alkaline-earth metal salts and alkali metal and alkaline-earth metal hydrogen carbonates, and mixtures thereof.

**47.** Process according to Claim 44 or 45, in which the component (C) is a naturally alkaline mineral water.

**48.** Process according to Claim 44, in which the organic base is chosen from alkanolamines.

**49.** Process according to any one of Claims 1 to 48, in which the component (C) is packaged in the form of a bottle, a jar, a tube, a sachet, a wipe, an aerosol, a spray or a solid stick.

**Patentansprüche**

**1.** Verfahren zum Färben von Keratinsubstanzen, **dadurch gekennzeichnet, dass**

(i) in einem ersten Schritt in Gegenwart von Sauerstoff, beispielsweise Luftsauerstoff, auf den keratinischen Träger eine einzige Komponente (A) aufgebracht wird, die in einem physiologisch akzeptablen Medium mindestens ein Farbstoffvorprodukt eines Farbstoffes, das unter den Verbindungen ausgewählt ist, die mindestens einen aromatischen Ring enthalten, der mindestens zwei Hydroxygruppen aufweist, die von zwei aufeinander folgenden Kohlenstoffatomen des aromatischen Rings getragen werden, und mindestens ein katalytisches System enthält, das einen ersten Katalysator (1), der unter den Salzen und Oxiden von Mn (II) und/oder Zn (II) und deren Gemischen ausgewählt ist, und einen zweiten Katalysator (2) enthält, der unter den Alkalihydrogencarbonaten, Erdalkalihydrogencarbonaten und deren Gemischen ausgewählt ist;
(ii) wenn die gewünschte Farbe erhalten wurde, auf den keratinischen Träger zur Fixierung der Farbe eine Komponente (B) aufgebracht wird, die eine saure Zusammensetzung enthält;
(iii) dann auf den keratinischen Träger eine Komponente (C) aufgebracht wird, die eine alkalische Zusammensetzung enthält, um die Farbintensität zu erhöhen oder die Färbung zu modifizieren,
(iv) wobei die erhaltene neue Farbe nochmals fixiert werden kann, indem die Zusammensetzung (B) erneut aufgebracht wird.

**2.** Verfahren nach Anspruch 1, **dadurch gekennzeichnet, dass**

(i) in einem ersten Schritt eine Komponente ($A_1$) auf den keratinischen Träger aufgebracht wird, die mindestens ein Farbstoffvorprodukt eines Farbstoffes und einen der Katalysatoren (1) und (2) enthält, wie sie in Anspruch 1 definiert wurden, und dann in einem zweiten Schritt die Farbe in Gegenwart von Sauerstoff, beispielsweise Luftsauerstoff, gebildet wird, indem eine Komponente ($A_2$) aufgetragen wird, die den anderen Katalysator (1) oder (2) enthält;
(ii) wenn die gewünschte Farbe gebildet ist, auf den keratinischen Träger zur Fixierung der Farbe eine Komponente (B) aufgebracht wird, die eine saure Zusammensetzung enthält;
(iii) wenn die Farbintensität erhöht oder die Färbung modifiziert werden soll, dann eine Komponente (C), die eine alkalische Zusammensetzung enthält, auf den keratinischen Träger aufgebracht wird;
(iv) wobei die erhaltene neue Farbe nochmals fixiert werden kann, indem die Zusammensetzung (B) erneut

aufgetragen wird.

3. Verfahren zum Färben von Keratinsubstanzen, **dadurch gekennzeichnet, dass**

(i) in einem ersten Schritt eine einzige Komponente (A), wie sie in Anspruch 1 definiert wurde, in Gegenwart von Sauerstoff, beispielsweise Luftsauerstoff, auf den keratinischen Träger aufgebracht wird;
(ii) auf den keratinischen Träger dann eine Komponente (C) aufgebracht wird, die eine alkalische Zusammensetzung umfasst, um die Farbintensität zu erhöhen oder die Färbung zu modifizieren;
(iii) wobei die erhaltene neue Farbe fixiert werden kann, indem eine Komponente (B) aufgebracht wird, die eine saure Zusammensetzung umfasst.

4. Verfahren nach Anspruch 3, **dadurch gekennzeichnet, dass**:

(i) in einem ersten Schritt eine Komponente ($A_1$), die mindestens ein Farbstoffvorprodukt eines Farbstoffes und einen der Katalysatoren (1) oder (2) enthält, wie sie in Anspruch 1 definiert wurden, auf den keratinischen Träger aufgebracht wird und dann in einem zweiten Schritt die Farbe in Gegenwart von Sauerstoff, beispielsweise Luftsauerstoff, gebildet wird, indem eine Komponente ($A_2$) aufgebracht wird, die den anderen Katalysator (1) oder (2) enthält;
(ii) eine Komponente (C), die eine alkalische Zusammensetzung enthält, auf den keratinischen Träger aufgebracht wird;
(iii) wobei die erhaltene neue Farbe fixiert werden kann, indem eine Komponente (B) aufgebracht wird, die eine saure Zusammensetzung enthält.

5. Verfahren nach einem der Ansprüche 1 bis 4, wobei in der Komponente (A) oder in den Komponenten ($A_1$) und ($A_2$) die Anteile des ersten Katalysators (1) und des zweiten Katalysators (2) so gewählt sind, dass:

$$\frac{[Mn(II)]}{[HCO_3]} \leq 1 \text{ mit } [\mathrm{Mn(II)}] \neq 0$$

$$\frac{[Zn(II)]}{[HCO_3]} \leq 1 \text{ mit } [\mathrm{Zn(II)}] \neq 0$$

$$\frac{[Mn(II) + Zn(II)]}{[HCO_3]} \leq 1 \text{ mit } [\mathrm{Mn(II)}] \text{ und } [\mathrm{Zn(II)}] \neq 0$$

worin [Mn(II)], [Zn(II)] und [$HCO_3$] die jeweiligen molaren Konzentrationen an Mn(II), Zn(II) und $HCO_3$ in der Zusammensetzung bedeuten.

6. Verfahren nach Anspruch 5, **dadurch gekennzeichnet, dass** das Verhältnis $\frac{[Mn(II)]}{[HCO]_3}$ im Bereich von $10^{-5}$ bis $10^{-1}$, vorzugsweise $10^{-3}$ bis $10^{-2}$ und noch besser in der Größenordnung von $5 \cdot 10^{-3}$ liegt.

7. Verfahren nach Anspruch 5 oder 6, **dadurch gekennzeichnet, dass** das Verhältnis $\frac{[Zn(II)]}{[HCO]_3}$ im Bereich von $10^{-4}$ bis < 1, vorzugsweise $10^{-3}$ bis < 1 und noch besser in der Größenordnung von $5 \cdot 10^{-1}$ liegt.

8. Verfahren nach einem der Ansprüche 5 bis 7, **dadurch gekennzeichnet, dass** das Verhältnis

$$\frac{[Mn(II) + Zn(II)]}{[HCO_3]}$$

im Bereich von $10^{-5}$ bis $10^{-1}$ und vorzugsweise $10^{-3}$ bis $10^{-2}$ liegt.

**9.** Verfahren nach einem der Ansprüche 1 bis 8, **dadurch gekennzeichnet, dass** die Salze von Mn(II) und Zn(II) der Komponente (A) oder der Komponenten ($A_1$) und ($A_2$) unter den Chloriden, Fluoriden, Iodiden, Sulfaten, Phosphaten, Nitraten, Perchloraten, Salzen von Carbonsäuren und deren Gemischen ausgewählt sind.

**10.** Verfahren nach Anspruch 9, **dadurch gekennzeichnet, dass** es sich bei dem Mn(II)-Salz oder Zn(II)-Salz um das Chlorid handelt.

**11.** Verfahren nach Anspruch 9, **dadurch gekennzeichnet, dass** die Carbonsäuresalze Salze von hydroxylierten Carbonsäuren sind.

**12.** Verfahren nach Anspruch 11, **dadurch gekennzeichnet, dass** das hydroxylierte Carbonsäuresalz das Gluconat ist.

**13.** Verfahren nach einem der Ansprüche 1 bis 12, **dadurch gekennzeichnet, dass** das Hydrogencarbonat unter Natriumhydrogencarbonat, Kaliumhydrogencarbonat, Magnesiumhydrogencarbonat, Calciumhydrogencarbonat und deren Gemischen ausgewählt ist.

**14.** Verfahren nach einem der Ansprüche 1 bis 13, **dadurch gekennzeichnet, dass** der aromatische Ring, der mindestens zwei Hydroxygruppen an zwei aufeinander folgenden Kohlenstoffatomen aufweist, an dem Farbstoffvorprodukt des Farbstoffes ein Benzolring oder ein kondensierter aromatischer Ring ist.

**15.** Verfahren nach Anspruch 14, **dadurch gekennzeichnet, dass** das Farbstoffvorprodukt des Farbstoffes eine Verbindung der folgenden Formel ist:

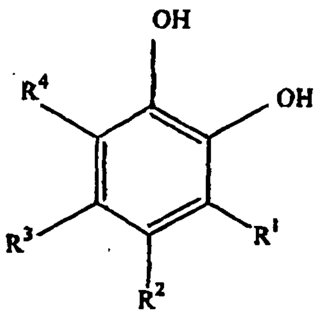

(I)

wobei die Substituenten $R^1$ bis $R^4$, die gleich oder verschieden sind, ein Wasserstoffatom, Halogen, Hydroxy, Carboxy, Alkylcarboxylat, eine gegebenenfalls substituierte Aminogruppe, eine geradkettige oder verzweigte, gegebenenfalls substituierte Alkylgruppe, eine geradkettige oder verzweigte, gegebenenfalls substituierte Alkenylgruppe, eine gegebenenfalls substituierte Cycloalkylgruppe, Alkoxy, Alkoxyalkyl, Alkoxyaryl, wobei die Arylgruppe gegebenenfalls substituiert ist, Aryl, eine substituierte Arylgruppe, eine gegebenenfalls substituierte heterocyclische Gruppe, eine Gruppe, die gegebenenfalls ein oder mehrere Siliciumatome enthält, bedeuten, oder wobei zwei der Substituenten $R^1$ bis $R^4$ gemeinsam einen gesättigten oder ungesättigten Ring bilden können, der gegebenenfalls ein oder mehrere Heteroatome enthält und gegebenenfalls mit einem oder mehreren gesättigten oder ungesättigten Ringen kondensiert ist, die gegebenenfalls ein oder mehrere Heteroatome enthalten.

**16.** Verfahren nach einem der Ansprüche 1 bis 13, **dadurch gekennzeichnet, dass** das Farbstoffvorprodukt des Farbstoffes unter den Flavanolen, Flavonolen, Anthocyanidinen, Anthocyaninen, Hydroxybenzoaten, Flavonen, Iridoiden, Verbindungen, die gegebenenfalls zuckerhaltig sein und/oder in Form von Oligomeren vorliegen können, gegebenenfalls Zucker enthaltenden Hydroxystilbenen, 3,4-Dihydroxyphenylalanin und seinen Derivaten, 2,3-Dihydroxyphenylalanin und seinen Derivaten, 4,5-Dihydroxyphenylalanin und seinen Derivaten, 4,5-Dihydroxyindol und seinen Derivaten, 5,6-Dihydroxyindol und seinen Derivaten, 6,7-Dihydroxyindol und seinen Derivaten, 2,3-Dihydroxyindol und seinen Derivaten, Dihydroxycinnamaten, Hydroxycumarinen, Hydroxyisocumarinen, Hydroxycumaronen, Hydroxyisocumaronen, Hydroxychalconen, Hydroxychromonen, Anthocyanen, Chinonen, Hydroxyxantonen und Gemischen aus zwei oder mehreren der vorhergehenden Verbindungen ausgewählt ist.

**17.** Verfahren nach einem der Ansprüche 1 bis 16, **dadurch gekennzeichnet, dass** das Farbstoffvorprodukt des Farbstoffes unter den Pflanzenextrakten, Fruchtextrakten, Agrumenextrakten, Gemüseextrakten und deren Gemischen ausgewählt ist.

**18.** Verfahren nach Anspruch 17, **dadurch gekennzeichnet, dass** das Farbstoffvorprodukt des Farbstoffes unter den Extrakten aus Tee, Traube, Apfel, Kakao, Sorghum, Banane, Kartoffel und deren Gemischen ausgewählt ist.

**19.** Verfahren nach einem der Ansprüche 1 bis 18, **dadurch gekennzeichnet, dass** das Farbstoffvorprodukt des Farbstoffes in der fertigen Komponente (A) in einer Menge von mindestens 10 Mikromol pro Milliliter Komponente (A) enthalten ist.

**20.** Verfahren nach einem der Ansprüche 1 bis 18, **dadurch gekennzeichnet, dass** das physiologisch akzeptable Medium der Komponente (A) oder der Komponenten ($A_1$) und ($A_2$) ein Medium ist, dass das Farbstoffvorprodukt des Farbstoffes löst, vorzugsweise mit bakteriostatischen Eigenschaften.

**21.** Verfahren nach einem der Ansprüche 1 bis 20, **dadurch gekennzeichnet, dass** das physiologisch akzeptable Medium der Komponente (A) oder der Komponenten ($A_1$) und ($A_2$) ein Lösungsmittel oder ein Gemisch von Lösungsmitteln für das Farbstoffvorprodukt des Farbstoffes enthält.

**22.** Verfahren nach Anspruch 21, **dadurch gekennzeichnet, dass** das Lösungsmittel unter Wasser, Alkoholen, Ethern, Dimethylsulfoxid, N-Methylpyrrolidon, Acetonen und deren Gemischen ausgewählt ist.

**23.** Verfahren nach Anspruch 22, **dadurch gekennzeichnet, dass** der Alkohol ein Alkanol oder ein Alkandiol ist.

**24.** Verfahren nach Anspruch 22, **dadurch gekennzeichnet, dass** das Lösungsmittel ein Gemisch Wasser/Alkohol ist.

**25.** Verfahren nach Anspruch 24, **dadurch gekennzeichnet, dass** der Alkohol bis zu 80 Gew.-% des Gemisches, vorzugsweise 1 bis 50 Gew.-% und besser 5 bis 20 Gew.-% ausmacht.

**26.** Verfahren nach einem der Ansprüche 1 bis 25, **dadurch gekennzeichnet, dass** die Komponente (A) keinen Chelatbildner für das Mn(II)-Salz und/oder Zn(II)-Salz enthält.

**27.** Verfahren nach einem der Ansprüche 1 bis 26, **dadurch gekennzeichnet, dass** die Komponente (A) kein pigmentierungsförderndes Enzym enthält.

**28.** Verfahren nach einem der Ansprüche 1 bis 27, wobei die Komponente (A) in Einzelform oder die Komponenten ($A_1$) und ($A_2$) als Creme, Milch, Gel, Gelcreme, Lotion, Pulver oder fester Block vorliegen.

**29.** Verfahren nach einem der Ansprüche 1 bis 28, wobei die Komponente (A) in einer Vorrichtung mit nur einer Abteilung konfektioniert ist, worin sich das oder die Farbstoffvorprodukte des Farbstoffes und das katalytische System befinden.

**30.** Verfahren nach Anspruch 29, wobei die Vorrichtung unter den luftdicht abgeschlossenen Metalltuben, Ampullen, Beuteln, versiegelten Tüchern, Aerosolen, die ein oder mehrere herkömmliche inerte Treibgase enthalten, Pumpvorrichtungen ohne Luftzufuhr oder festen Blöcken ausgewählt ist.

**31.** Verfahren nach einem der Ansprüche 1 bis 30, wobei die beiden Komponenten ($A_1$) und ($A_2$) als Kit konfektioniert sind, das zwei unterschiedliche Abteilungen aufweist; wobei die erste Abteilung die Komponente ($A_1$), die das oder die Farbstoffvorprodukte des Farbstoffes und einen der Katalysatoren (1) oder (2) enthält, wie sie in den vorhergehenden Ansprüchen definiert wurden, und die zweite Abteilung die Komponente ($A_2$) enthält, die den anderen Katalysator (1) oder (2) enthält, wobei die Komponenten ($A_1$) und ($A_2$) bei der Anwendung vermischt werden oder nacheinander aufgetragen werden.

**32.** Verfahren nach Anspruch 31, wobei jede Abteilung unabhängig von der anderen in einer Vorrichtung konfektioniert ist, die unter den luftlicht abgeschlossenen Metalltuben, Ampullen, Beuteln, versiegelten Tüchern, Aerosolen, die

ein oder mehrere herkömmliche inerte Treibgase enthalten, Pumpvorrichtungen ohne Luftzufuhr oder festen Blöcken ausgewählt ist.

**33.** Verfahren nach Anspruch 31, wobei die Vorrichtung ein Aerosol mit zwei Abteilungen ist, die die Komponenten ($A_1$) bzw. ($A_2$) enthalten und mit denen selektiv eine Abgabeöffnung verbunden werden kann; wobei die Komponenten ($A_1$) und ($A_2$) bei der Anwendung in Abhängigkeit von der Konfiguration der Vorrichtung gleichzeitig oder nacheinander abgegeben werden können.

**34.** Verfahren nach Anspruch 31, wobei die Vorrichtung ein System mit zwei Abteilungen ist, die jeweils mit einer Pumpe ohne Luftzufuhr ausgestattet sind, wobei die erste Abteilung die Komponente ($A_1$) und die andere Abteilung die Komponente ($A_2$) enthält, wobei die Komponenten ($A_1$) und ($A_2$) bei der Anwendung in Abhängigkeit von der Konfiguration der Vorrichtung gleichzeitig oder nacheinander abgegeben werden können.

**35.** Verfahren nach einem der Ansprüche 1 bis 34, wobei die Komponente (A) oder die Komponenten ($A_1$) und ($A_2$) in Form von in Wasser spaltbaren festen Blöcken vorliegen.

**36.** Verfahren nach einem der Ansprüche 1 bis 35, wobei die Komponente (B) eine wässrige Zusammensetzung umfasst, die mindestens eine anorganische oder organische Säure enthält.

**37.** Verfahren nach Anspruch 36, wobei der pH-Wert der Komponente (B) im Allgemeinen im Bereich von 1 bis 6 und vorzugsweise 2 bis 5 liegt.

**38.** Verfahren nach Anspruch 36 oder 37, wobei die anorganische Säure unter Salzsäure (HCl), Phosphorsäure ($H_3PO_4$) oder deren Gemischen ausgewählt ist.

**39.** Verfahren nach Anspruch 36 oder 37, wobei die Komponente (B) aus einem natürlich sauren Wasser besteht.

**40.** Verfahren nach Anspruch 36 oder 37, wobei die organische Säure unter Essigsäure, $\alpha$-Hydroxysäuren, $\beta$-Hydroxysäuren, $\alpha$- und $\beta$-Ketosäuren oder deren Gemischen ausgewählt ist.

**41.** Verfahren nach Anspruch 36 oder 37, wobei die organische Säure unter Glycolsäure, Milchsäure, Äpfelsäure, Weinsäure, Citronensäure, Mandelsäure, Salicylsäure oder deren alkylierten oder alkoxylierten Derivaten und deren Gemischen ausgewählt ist.

**42.** Verfahren nach Anspruch 41, wobei die organische Säure unter Milchsäure, Glycolsäure, Citronensäure und deren Gemischen ausgewählt ist.

**43.** Verfahren nach einem der Ansprüche 1 bis 42, wobei die Komponente (B) als Flakon, Tiegel, Tube, Beutel, Tuch, Aerosol, Spray oder fester Stift konfektioniert ist.

**44.** Verfahren nach einem der Ansprüche 1 bis 43, wobei die Komponente (C) eine wässrige Zusammensetzung umfasst, die mindestens eine anorganische Base und/oder organische Base enthält.

**45.** Verfahren nach Anspruch 44, wobei der pH-Wert der Komponente (C) im Allgemeinen im Bereich von 7 bis 12 und vorzugsweise 8 bis 10 liegt.

**46.** Verfahren nach Anspruch 44, wobei die anorganische Base unter den Alkalimetallsalzen oder Erdalkalimetallsalzen; Alkalihydrogencarbonaten und Erdalkalihydrogencarbonaten und deren Gemischen ausgewählt ist.

**47.** Verfahren nach Anspruch 44 oder 45, wobei die Komponente (C) ein natürlich alkalisches Mineralwasser ist.

**48.** Verfahren nach Anspruch 44, wobei die organische Base unter den Alkanolaminen ausgewählt ist.

**49.** Verfahren nach einem der Ansprüche 1 bis 48, wobei die Komponente (C) als Flakon, Tiegel, Tube, Beutel, Tuch, Aerosol, Spray oder fester Stift konfektioniert ist.